# EUROPÄISCHE PATENTSCHRIFT

(11) **EP 1 272 059 B1**
(45) Veröffentlichungstag und Bekanntmachung des Hinweises auf die Patenterteilung: **07.12.2005**
(21) Anmeldenummer: 01927730.0
(22) Anmeldetag: 15.03.2001
(51) Int. Cl.: A23L 1/302, A61K 9/10, A61K 31/525, A61K 31/4415, A61K 31/593, A61K 31/375, A61K 31/714

(54) **VERFAHREN ZUR HERSTELLUNG ÖLIGER SUSPENSIONEN WASSERLÖSLICHER VITAMINE**
METHOD OF PRODUCING OILY SUSPENSIONS OF WATER-SOLUBLE VITAMINS
PROCEDE POUR LA PRODUCTION DE SUSPENSIONS HUILEUSES DE VITAMINES SOLUBLES DANS L'EAU

(30) Priorität: 17.03.2000 DE 10013312; 04.10.2000 DE 10049137
(43) Veröffentlichungstag der Anmeldung: 08.01.2003
(73) Patentinhaber: BASF Aktiengesellschaft, 67056 Ludwigshafen (DE)
(72) Erfinder: BETZ, Roland, 67150 Niederkirchen (DE); BEWERT, Wolfgang, 67227 Frankenthal (DE); ERNST, Andreas, 67551 Worms (DE); KÄSLER, Bruno, 67071 Ludwigshafen (DE); PFEIFFER, Angelika-Maria, 67134 Birkenheide (DE); RUNGE, Frank, 67159 Friedelsheim (DE); SCHNEIDER, Joachim, U., 67273 Weisenheim (DE); STANG, Michael, 67069 Ludwigshafen (DE)
(86) Internationale Anmeldenummer: PCT/EP2001/002939
(87) Internationale Veröffentlichungsnummer: WO 2001/067896

(56) Entgegenhaltungen:
- EP-A- 0 772 978
- WO-A-98/00038
- US-A- 1 358 401

## Beschreibung

Die Erfindung betrifft ölige Suspensionen mindestens eines wasserlöslichen Vitamins sowie ein Verfahren zur Herstellung dieser Suspensionen und deren Verwendung als Zusatz zu Lebensmitteln, Tierfuttermitteln, Pharmazeutika und kosmetischen Zubereitungen.

Die Verwendung von Vitaminen als Futtermittelzusatzstoffe in der Tierernährung erfolgt mehr und mehr in Form flüssiger Zubereitungen. Dies hat u.a. den Vorteil, daß die Dosierung einfacher und genauer erfolgen kann. Ferner ist es möglich, bei der sogenannten "post-pelleting-application" beispielsweise Futtermittelpellets erst nach deren Herstellung mit einer flüssigen Zubereitung von Futtermittelzusatzstoffen zu beladen. Dies hat zur Folge, daß selbst oxidations- und temperaturempfindliche Zusatzstoffe wie Vitamine oder Carotinoide ohne größere Verluste eingesetzt werden können.

Beispiele für "post-pelleting-application" (PPA) finden sich u.a. in GB-A-2 .232 573 sowie in EP-A-0 556 883 und der darin zitierten Literatur.

Eine bislang noch kritische Anwendung ist die Verabreichung flüssiger Formulierungen von wasserlöslichen Vitaminen in Kombination mit Mineralien und fettlöslichen Vitaminen. Hier kann es durch gegenseitige Wechselwirkungen zu unerwünschten Wirkstoffverlusten kommen.

EP-A-0 772 978 beschreibt eine Mischung derartiger Substanzen, bei denen die Vitamine und Mineralien separat aufbewahrt und erst kurz vor der Applikation gemischt werden. Nachteilig bei dieser Vorgehensweise ist die erforderliche Bereitstellung entsprechender Lagerbehälter, verbunden mit einer aufwendigen Logistik.

Vitamin-Emulsionen - als spezielle Form einer flüssigen Formulierung - haben häufig den Nachteil, daß sie physikalisch (Auftreten von Phasentrennung) und chemisch (Auftreten von unerwünschten Hydrolyse- und/oder Redox-Reaktionen, chemische Inkompatibilität einzelner gelöster Komponenten) instabil sind und zusätzlich häufig die Gefahr einer mikrobiologischen Kontamination auftreten kann.

Ferner sind Systeme bekannt, bei denen u.a. wasserlösliche Vitamine in Ölen oder Fetten dispergiert vorliegen.

So beschreibt GB-A-1 358 401 die Herstellung von Lebensmittelsupplementen basierend auf eßbaren Hartfetten mit Schmelzpunkten von 37 bis 121°C. Den geschmolzenen Hartfetten werden dabei unter Rühren feinverteilte assimilierbare eisenhaltige Substanzen zugegeben. Die homogene Dispersion wird anschließend zu Hartfettpellets sprühgekühlt. Optionell können zudem auch Mikronährstoffe und Vitamine eingearbeitet werden. Eine Anwendung in flüssiger Form ist mit den hier genannten Hartfetten nur bei erhöhter Temperatur möglich, was verfahrensstechnisch aufwendig ist und bei der Verwendung von Vitaminen häufig mit der Gefahr unerwünschter Wirkstoffverluste verbunden ist.

WO 98/00038 offenbart ölige suspensionen wasserlösslicher Vitamine.

Es war daher die Aufgabe der vorliegenden Erfindung, stabile flüssige Formulierungen wasserlöslicher Vitamine bereitzustellen, die die oben genannten Nachteile des Standes der Technik nicht aufweisen.

Diese Aufgabe wurde erfindungsgemäß gelöst durch ein Verfahren zur Herstellung öliger Suspensionen wasserlöslicher Vitamine, dadurch gekennzeichnet, daß man
a) mindestens ein wasserlösliches Vitamin in einem Öl, bevorzugt in mindestens einem eßbaren Öl bis auf eine mittlere Partikelgröße von 0,1 bis 100 µm mahlt oder
b) mindestens ein wasserlösliches Vitamin ohne Verwendung einer kontinuierlichen Phase bis auf eine mittlere Partikelgröße von 0,1 bis 100 µm mahlt und die gemahlenen Partikel anschließend in einem Öl, bevorzugt in mindestens einem eßbaren Öl suspendiert.

Bei den wasserlöslichen Vitaminen handelt es sich insbesondere um Ascorbinsäure und deren Salze wie Natriumascorbat sowie Vitamin C-Derivate wie Natrium-, Calcium- oder Magnesium-ascorbyl-2-monophosphat oder Calcium-ascorbyl-2-polyphosphat, Calcium-pantothenat, Panthenol, Vitamin B₁ (Thiamin) - als Hydrochlorid, Nitrat oder Pyrophosphat, Vitamin B₂ (Riboflavin) und deren Phosphate, Vitamin B₆ und Salze, Vitamin B₁₂, Biotin, Folsäure und Folsäurederivate wie Tetrahydrofolsäure, 5-Methyltetrahydrofolsäure, 5-Formyltetrahydrofolsäure, Nicotinsäure und Nicotinsäureamid.

Als wasserlösliches Vitamin sei in diesem Zusammenhang auch Vitamin K₃ (Menadion) als Natriumbisulfit genannt.

Die oben genannten wasserlöslichen Vitamine können sowohl in kristalliner Form mit einer Reinheit größer 90 %, bevorzugt größer 95 %, besonders bevorzugt größer 98 %, als auch in formulierter Form, beispielsweise als Granulat, Beadlet oder als sprühgetrocknetes Pulver eingesetzt werden. Bevorzugt sind die o.g. Vitamine in ihrer kristallinen Form.

Als eßbare Öle kommen in der Regel alle physiologisch unbedenklichen Öle - sowohl pflanzlichen als auch tierischen Ursprungs - in Frage, insbesondere solche Öle, die bei 20°C flüssig sind bzw. die in der Suspension bei 20°C allein oder zusammen mit anderen Ölen die flüssige Phase bilden. Bevorzugt zu nennen sind in diesem Zusammenhang Sonnenblumenöl, Palmöl, Sesamöl, Maiskeimöl, Baumwollsaatöl, Sojabohnenöl oder Erdnußöl, Ester mittelkettiger Triglyceride sowie außerdem Fischöle wie beispielsweise Makrelen-, Sprotten- oder Lachsöl. Für die Tierernährung besonders bevorzugt sind Fischöle, Maiskeimöl, Sonnenblumenöl und Erdnußöl. Für den Food-/Pharmabereich zusätzlich von Vorteil sind die Ester mittelkettiger Triglyceride.

Als eßbares Öl im Sinne der Erfindung sind auch Vitamin E, Vitamin E-Derivate oder Mischungen davon zu verstehen. Die Bezeichnung,Vitamin E steht in diesem Zusammenhang für natürliches oder synthetisches α-, β-, γ- oder δ-Tocopherol, bevorzugt für natürliches oder synthetisches α-Tocopherol sowie für Tocotrienol. Vitamin E-Derivate sind z.B. Tocopheryl-C₁-C₂₀-Alkansäureester wie Tocopherylacetat oder Tocopherylpalmitat.

Vitamin E und/oder deren Derivate können dabei allein oder zusammen mit den anderen eßbaren Ölen als Dispergiermedium verwendet werden.

Die Mahlung kann in an sich bekannter Weise z.B. mit einer Kugelmühle erfolgen. Dabei wird je nach verwendetem Mühlentyp so lange gemahlen, bis die Teilchen eine über Fraunhofer Beugung ermittelte mittlere Partikelgröße D[4,3] von 0,1 bis 100 µm, bevorzugt 0,2 bis 50 µm, besonders bevorzugt 0,5 bis 30 µm, ganz besonders bevorzugt 0,8 bis 20 µm, insbesondere 1,0 bis 10 µm aufweisen. Der Begriff D[4,3] bezeichnet den volumengewichteten mittleren Durchmesser (siehe Handbuch zu Malvern Mastersizer S, Malvern Instruments Ltd., UK).

Nähere Einzelheiten zur Mahlung und den dafür eingesetzten Apparaturen finden sich u.a. in Ullmann's Encyclopedia of Industrial Chemistry, Sixth Edition, 1999, Electronic Release, Size Reduction, Kapitel 3.6.: Wet Grinding.

Bei dem erfindungsgemäßen Mahlverfahren ist es möglich, alle in der Suspension verwendeten Komponenten aus der Gruppe der wasserlöslichen Vitamine als Gesamtmischung zu mahlen. Es kann aber auch jede einzelne zu mahlende Komponente in hoher Konzentration in dem zu verwendenden Öl gemahlen werden. Die Endzubereitung ergibt sich dann durch eine Abmischung der jeweiligen Einzelsuspensionen.

Die erfindungsgemäße Zubereitung kann vor der Anwendung mit Fetten oder Ölen auf die jeweilige Gebrauchskonzentration verdünnt werden.

Eine besondere Ausführungsform des erfindungsgemäßen Verfahrens ist dadurch gekennzeichnet, daß die Mahlung im Schritt a) sowie die Mahlung und/oder Suspendierung im Schritt b) in Abwesenheit eines Emulgators erfolgen. Überraschenderweise wurde dabei festgestellt, daß sich auch ohne Zusatz eines Tensids feinteilige, homogene Suspensionen wasserlöslicher Vitamine in Ölen herstellen lassen, die auch in hohen Konzentrationen sedimentationsstabil sind.

Eine weitere vorteilhafte Ausführungsform des erfindungsgemäßen Verfahrens ist dadurch gekennzeichnet, daß die Mahlung im Schritt a) sowie die Mahlung und/oder Suspendierung im Schritt b) in Abwesenheit eines Schutzkolloids erfolgen.

Trotz Fehlen der o.g. Dispergier- bzw. Formulierhilfsmittel konnten die an sich hydrophilen Vitamine - für den Fachmann unerwartet - ohne Benetzungsprobleme und Agglomeratbildung in den o.g. hydrophoben Dispergiermedien feinstgemahlen werden.

Neben der oben beschriebenen Naßmahlung, lassen sich die erfindungsgemäßen öligen Suspensionen auch dadurch Trockenmahlung der wasserlöslichen Vitamine und anschließendes Suspendieren der gemahlenen Partikel in mindestens einem eßbaren Öl herstellen. Als Trockenmahlung versteht man in diesem Zusammenhang eine Mahlung ohne Verwendung einer kontinuierlichen Phase.

Nähere Einzelheiten zur Trockenmahlung finden sich u.a. in Ullmann's Encyclopedia of Industrial Chemistry, Sixth Edition, 1999, Electronic Release, Size Reduction, Kapitel 3.4.

Als besonderen Vorteil hinsichtlich der Stabilität der erfindungsgemäßen öligen Dispersionen hat sich herausgestellt, wenn man die Mahlung imVerfahrensschritt a) sowie die Mahlung und/oder Suspendierung im Schritt b) in Gegenwart von Trockenmitteln durchführt. Bevorzugt sind dabei Trockenmittel, ausgewählt aus der Gruppe, bestehend aus Alkali- und Erdalkalisulfaten wie Natrium-, Calcium- und Magnesiumsulfat, Alkali- und Erdalkalichloriden wie Natrium-, Calcium- und Magnesiumchlorid und Kieselgel. Als ganz besonders bevorzugtes Trockenmittel ist CaCl₂ zu nennen.

Die Menge an eingesetztem Trockenmittel liegt im allgemeinen zwischen 0,1 und 20 Gew.-%, bevorzugt zwischen 0,5 und 15 Gew.-%, besonders bevorzugt zwischen 1,0 und 10 Gew.-%, bezogen auf die Gesamtmenge der öligen Suspension.

Das oder die verwendeten Trockenmittel können dabei auch separat - wie im Verfahrensschritt a) - in einem eßbaren Öl gemahlen werden und anschließend der öligen Suspension der gemahlenen wasserlöslichen Vitamine zugesetzt werden. Ferner ist es möglich, die Trockenmittel auch ungemahlen mit der öligen Suspension der gemahlenen wasserlöslichen Vitamine aus Verfahrensschritt a) zu mischen. Im Falle der Trockenmahlung können die wasserlöslichen Vitamine und das oder die Trockenmittel auch separat gemahlen und dann zur öligen Suspension gegeben werden.

Aufgrund der feinteiligen Verteilung der dispergierten wasserlöslichen Vitamine zeichnen sich die nach dem erfindungsgemäßen Verfahren hergestellten öligen Suspensionen durch eine hohe Bioverfügbarkeit der in der Suspension enthaltenen Wirkstoffe aus.

Neben den eingangs genannten wasserlöslichen Vitaminen können vor, während oder nach der Mahlung zusätzliche fettlösliche Vitamine, wie z.B. die K-Vitamine, Vitamin A und Derivate wie Vitamin A-Acetat, Vitamin A-Propionat oder Vitamin A-Palmitat, Vitamin D₂ und Vitamin D₃ sowie die bereits genannten E-Vitamine in die ölige Suspension eingetragen und gelöst werden. Bevorzugt erfolgt die Mahlung im Schritt a) sowie die Suspendierung im Schritt b) in Gegenwart fettlöslicher Vitamine.

Gegenstand der Erfindung sind auch ölige Suspensionen mindestens eines wasserlöslichen Vitamins, erhältlich nach einem Verfahren, das dadurch gekennzeichnet ist, daß man mindestens ein wasserlösliches Vitamin in mindestens einem eßbaren Öl bis auf eine mittlere Partikelgröße von 0,1 bis 100 µm mahlt.

Die erfindungsgemäßen öligen Suspensionen enthalten dabei 5 bis 70 Gew.-%, bevorzugt 5 bis 60 Gew.-%, besonders bevorzugt 10 bis 55 Gew.-% ganz besonders bevorzugt 15 bis 50 Gew.-% mindestens eines der eingangs genannten wasserlöslichen Vitamine in feinstvermahlener Form.

Außerdem können die öligen Suspensionen zusätzlich 0,5 bis 60 Gew.-%, bevorzugt 5 bis 50 Gew.-%, besonders bevorzugt 10 bis 45 Gew.-%, ganz besonders bevorzugt 15 bis 40 Gew.-% mindestens eines der eingangs genannten fettlöslichen Vitamine in gelöster Form enthalten.

Darüberhinaus können die öligen Zubereitungen zusätzlich mindestens ein weiteres Carotinoid enthalten.

Unter Carotinoide sind z.B. folgende Verbindungen zu verstehen: β-Carotin, Lycopin, Lutein, Astaxanthin, Zeaxanthin, Cryptoxanthin, Citranaxanthin, Canthaxanthin, Bixin, β-Apo-4-carotinal, β-Apo-8-carotinal, β-Apo-8-carotinsäureester, einzeln oder als Mischung. Bevorzugt verwendete Carotinoide sind β-Carotin, Lycopin, Lutein, Astaxanthin, Zeaxanthin, Citranaxanthin und Canthaxanthin.

Die Carotinoide können dabei in kristalliner Form oder als Formulierung - beispielsweise als Trockenpulver, gemäß EP-A-0 065 193 eingesetzt werden.

Vorteilhafterweise werden die Carotinoide in der Regel in kristalliner Form zusammen mit den wasserlöslichen Vitaminen in dem Öl gemahlen. Im Falle von Astaxanthin und Canthaxanthin werden bevorzugt Astaxanthin- bzw. Canthaxanthin-haltige Trockenpulver, beispielsweise Lucantin® Pink bzw. Lucantin® Rot (ein 10%iges Astaxanthin- bzw. Canthaxanthin-Trockenpulver, Fa. BASF Aktiengesellschaft, Ludwigshafen, Deutschland) zusammen mit den wasserlöslichen Vitaminen eingesetzt.

Der Gehalt an Carotinoide liegt in den Formulierungen im allgemeinen zwischen 0,1 und 40 Gew.-%, bevorzugt zwischen 0,3 und 20 Gew.-%, besonders bevorzugt zwischen 0,5 und 10 Gew.-%, ganz besonders bevorzugt zwischen 1 und 5 Gew.-%, bezogen auf die Gesamtmenge der Formulierung.

Je nach Anwendungszweck können die erfindungsgemäßen öligen Zubereitungen bis zu 10 Gew.-% weiterer Zusatzkomponenten wie beispielsweise Mineralstoffe, Aminosäuren, Proteine oder Enzyme enthalten.

Diese Zusatzstoffe, ebenso wie die o.g. fettlöslichen Vitamine und Carotinoide, können vor, während oder nach der Mahlung der erfindungsgemäßen Suspension zugesetzt werden. Um eine möglichst feinteilige homogene Suspension aller nicht-öllöslicher Bestandteile zu erhalten, ist es von Vorteil, die o.g. Zusatzstoffe ebenfalls zusammen mit den wasserlöslichen Vitaminen zu mahlen.

Als Mineralstoffe können beispielsweise Eisensulfat, Zinksulfat, Mangansulfat, Kupfersulfat, Calciumsulfat, Natriumsulfat, Kupferoxid, Magnesiumoxid, Calciumfluorid, Kaliumchlorid, Kaliumjodid, Natriumchlorid, Calciumjodat, Calcium-, Magnesium-, Kalium-, Natrium- oder Eisen-Phosphat, Cobaltcarbonat, Natriumselenat oder Kieselsäure und deren Salze in die Suspension eingearbeitet und mitvermahlen werden. Die Menge an eingesetzten Mineralstoffen, beispielsweise im Tierernährungsbereich, orientiert sich dabei jeweils am Bedarf der zu fütternden Tiere.

Als Aminosäurereste kommen generell alle bekannten physiologisch unbedenklichen α-Aminosäurereste in Frage. Bevorzugt zu nennen sind die Reste folgender Aminosäuren: Alanin, Arginin, Asparagin, Asparaginsäure, Cystein, Cystin, Glutamin, Glutaminsäure, Glycin, Histidin, Isoleucin, Leucin, Lysin, Methionin, Phenylalanin, Hippursäure, Serin und Taurin. Besonders bevorzugt sind Lysin, Methionin und Cystein.

Als Enzyme kommen in diesem Zusammenhang bevorzugt Phosphatasen Glucanasen und gegebenenfalls Esterasen bzw. Lipasen, letztere in verkapselter Form, in Frage.

Weitere Bestandteile der Suspension können sein:

Verbindungen mit Vitamin- oder Coenzymcharakter, z.B. Cholinchlorid, Carnitin, γ-Butyrobetain, Liponsäure, Kreatin, Ubichinone, S-Methylmethionin, S-Adenosylmethionin.

Mehrfach ungesättigte Fettsäuren, z.B. Linolsäure, Linolensäure, Arachidonsäure, Eicosapentaensäure, Docosahexaensäure.

Fütterungsantibiotika für Medizinalfutter sowie Mikroorganismen zur Verbesserung der Verdauung.

In manchen Fällen kann es erforderlich sein, daß die öligen Suspensionen außerdem Hilfsstoffe, wie z.B. Schutzkolloide, Antioxidantien, Verdicker, Chelatbildner, wie z.B. Alkali- oder Erdalkalisalze der Citronensäure, Phytinsäure oder Phosphorsäure enthalten und/oder Emulgatoren enthalten.

Als Schutzkolloide können beispielsweise Gelatine, Fischgelatine, Stärke, Dextrin, Pflanzenproteine, Pektin, Gummi-Arabikum, Kasein, Kaseinat oder Mischungen davon verwendet. Es können aber auch Polyvinylalkohol, Polyvinylpyrrolidon, Methylcellulose, Carboxymethylcellulose, Hydroxypropylcellulose und Alginate eingesetzt werden. Bezüglich näherer Einzelheiten wird auf R.A. Morton, Fat Soluble Vitamins, Intern. Encyclopedia of Food and Nutrition, Bd.9, Pergamon Press 1970, S. 128-131, verwiesen.

Zur Erhöhung der Stabilität des Wirkstoffes gegen oxidativen Abbau ist es vorteilhaft, Stabilisatoren wie α-Tocopherol, t-Butyl-hydroxy-toluol, t-Butylhydroxyanisol, Ascorbinsäure oder Ethoxyquin zuzusetzen.

Als Emulgatoren bzw. Solubilisatoren können beispielsweise Ascorbylpalmitat, Polyglycerin-Fettsäureester, Sorbitan-Fettsäureester, Propylenglycol-Fettsäureester oder Lecithin verwendet werden.

Die erfindungsgemäßen öligen Suspensionen haben u.a. den Vorteil, daß die Vitamine durch das Öl gegen Sauerstoff und Feuchtigkeit geschützt sind. Die wasserlöslichen Vitamine und die Mineralien sind nahezu ölunlöslich und können dadurch auch keine Reaktionen untereinander eingehen (Kompatibilität). Zudem sind mikrobiologische Probleme in öligen, wasserfreien Systemen nicht zu erwarten.

Die Suspensionen eignen sich u.a. als Zusatzstoff für Lebensmittel- und Tierfuttermittelzubereitungen bzw Mischfutter, als Mittel für die Herstellung pharmazeutischer und kosmetischer Zubereitungen sowie für die Herstellung von Nahrungsergänzungspräparaten im Human- und Tierbereich.

Bevorzugt lassen sich die Suspensionen als Futtermittelzusatz in der Tierernährung einsetzen, insbesondere zum Auftragen bzw. Aufsprühen auf Futtermittelpellets.

Die Anwendung als Futtermittelzusatzstoff erfolgt insbesondere durch direktes Aufsprühen der erfindungsgemäßen Suspensionen, gegebenenfalls nach Verdünnung mit Ölen, beispielsweise auf Tierfutterpellets als sogenannte "post-pelleting-application".

Eine bevorzugte Ausführungsform des Sprühverfahrens besteht darin, daß man die Futtermittelpellets unter vermindertem Druck mit der öligen Suspension belädt.

Beispiele hierfür finden sich u.a. in GB-A-2 232 573 sowie in EP-A-0 556 883.

Typische Einsatzgebiete im Lebensmittelbereich sind beispielsweise die Vitaminierung von Getränken, Milchprodukten wie Joghurt, Milchmixgetränken oder Milchspeiseeis sowie von Puddingpulvern, Eiprodukten, Backmischungen und Süßwaren.

Im Kosmetikbereich können die öligen Suspensionen beispielsweise für Vitamin-haltige Körperpflegemittel beispielsweise in Form einer Creme, einer Lotion, als Lippenstifte oder Make-up verwendet werden.

Bei den für kosmetische Zwecke verwendeten, erfindungsgemäßen Suspensionen wird die Ölphase vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstereat, Isopropyloleat, n-Butylstereat, N-Hexyllaurat, N-Decyloleat, Isooctylstearat, Isononylstereat, Isononylisnoanoat, 2-Ethylhexylpalmitat, 2-Ethylhexyllaurat, 2-Hexyldecylstereat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Siliconöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂-C₁₅-Alkylbenzoat, Capryl-Caprinsäure-triglycerid, Dicaprylether.

Besonders vorteilhaft sind Mischungen aus C₁₂-C₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisonanoat, aus Cyclomethicon und 2-Ethylhexylisostearat.

Gegenstand der Erfindung sind ferner Nahrungsergänzungsmittel, Tierfuttermittel, Lebensmittel sowie pharmazeutische und kosmetische Zubereitungen, enthaltend die eingangs beschriebenen öligen Suspensionen wasserlöslicher Vitamine.

Im Falle von pharmazeutischen Zubereitungen können die öligen Suspensionen folgende Wirkstoffe als Zusatzkomponente - einzeln oder im Gemisch - enthalten:
Schmerzmittel wie Acetylsalicylsäure, Ibuprofen, Flurbiprofen, Paracetamol, Propyphenazon,
Sympatomimetika wie Pseudoephedrin, Ephedrin, Phenylpropanolamin, Phenylephrin,
Antihistaminika wie Chlorpheniraminmaleat,
Antitussiva wie Dihydrocodein, Guaifenesin,
Pflanzenextrakte wie Weißdornextrakt, Bärentraubenbätterextrakt, Wacholderbeerenextrakt, Ginsengextrakt,
Coffein.

Bevorzugt richtet sich die Erfindung auf Tierfuttermittel, insbesondere auf Futtermittelpellets, die mit den Suspensionen beladen werden.

Unter Nahrungsergänzungspräparate sowie pharmazeutische Zubereitungen, die die erfindungsgemäße Suspension enthalten, sind u.a. Tabletten, Dragees sowie bevorzugt Hart- und Weichgelatinekapseln zu verstehen.

Kosmetische Zubereitungen, die die erfindungsgemäßen Suspensionen enthalten können, sind beispielsweise topisch anwendbare Zubereitungen, insbesondere dekorative Körperpflegemittel wie Lippenstifte, Gesichts-Make-up in Form einer Creme sowie Lotionen.

In den folgenden Beispielen wird die Herstellung der erfindungsgemäßen öligen Suspensionen wasserlöslicher Vitamine näher erläutert.

### Beispiel 1

Zwei Kilogramm einer Mischung aus 25 Gew.-% Vitamin C (99%ig, BASF Aktiengesellschaft) und 75 Gew.-% eines mittelkettigen Triglycerids (Delios® SK der Fa. Grünau, Deutschland) wurden mit einem Blattrührer solange gerührt, bis eine homogene Suspension vorlag. Danach wurde die Mischung in eine rührbare Vorlage umgefüllt, aus der die Suspension mittels Schlauchpumpe durch eine kontinuierlich betriebene Kugelmühle (Dyno Mill KDL Spezial) gefördert wurde. Der Mahlbehälter der Kugelmühle war mit 400 g Glaskugeln (Durchmesser 800 bis 1200 µm) gefüllt. Die aus der Mühle austretende feinteilige Suspension wurde aufgefangen und mittels eines Partikelgrößenmeßgerätes (Malvern Mastersizer) vermessen. Der Mahlvorgang wurde so oft wiederholt bis 90 % der suspendierten Teilchen eine Teilchengröße kleiner 10 µm [D(0.9) < 10 µm] hatten. Dies entsprach einer mittleren Partikelgröße D[4,3] von 5,2 µm.

Nach der Abtrennung von den Mahlkörpern wurde ein Teil der Dispersion mit der 10 fachen Menge des verwendeten Öls verdünnt und über 12 h stehengelassen. Weder die unverdünnte noch die verdünnte Dispersion zeigten über diesen Zeitraum Sedimentationserscheinungen.

### Beispiel 2

Eine Mischung aus

| Komponente | Menge in g |
|---|---|
| Vitamin C | 200 |
| Calcium-d-pantothenat | 140 |
| Nicotinsäure | 280 |
| Folsäure | 4,0 |
| Biotin | 2,0 |
| Vitamin B₁₂ . | 0,1 |
| Vitamin B₆-HCl | 20 |
| Vitamin B₂ | 100 |
| Vitamin B₁-HCl | 40 |
| Vitamin K₃ (Menadion-Na-Bisulfit) | 40 |
| Vitamin E-Acetat | 400 |
| Vitamin D₃ | 0,10 |
| Vitamin A-Acetat | 8,2 |
| D,1-α-Tocopherol | 600 |

wurde mit einem Blattrührer solange gerührt, bis eine homogene Suspension vorlag. Die Mischung wurde daraufhin in eine rührbare Vorlage umgefüllt, aus der die Suspension mittels Schlauchpumpe durch die in Beispiel 1 genannte, kontinuierlich betriebene Kugelmühle gefördert wurde. Der Mahlvorgang wurde so oft wiederholt bis 90 % der suspendierten Teilchen eine Teilchengröße kleiner 20 µm [D(0.9) < 20 µm] hatten. Dies entsprach einer mittleren Partikelgröße D[4,3] von 10,9 µm.

Nach der Abtrennung von den Mahlkörpern wurde ein Teil der Dispersion mit der 10 fachen Menge Maiskeimöl verdünnt und über 12 h stehengelassen. Weder die unverdünnte noch die verdünnte Dispersion zeigten über diesen Zeitraum Sedimentationserscheinungen.

### Beispiel 3

Eine Mischung aus

| Komponente | Menge in g |
|---|---|
| Vitamin C | 400 |
| Calcium-d-pantothenat | 140 |
| Nicotinsäure | 280 |
| Folsäure | 4,0 |
| Lutavit® H₂ (2%iges Biotin, Fa. BASF) | 100 |
| Vitamin B₁₂ | 0,1 |
| Vitamin B₆-HCl | 20 |
| Vitamin B₂ | 100 |
| Vitamin B₁-HCl | 40 |
| Vitamin K₃ (Menadion-Na-Bisulfit) | 20 |
| Vitamin E-Acetat | 400 |
| Vitamin D₃ | 0,10 |
| Vitamin A-Acetat | 8,2 |
| Maiskeimöl | 800 |

wurde analog Beispiel 2 suspendiert und durch eine kontinuierlich betriebene Kugelmühle gemahlen. Der Mahlvorgang wurde so lange durchgeführt, bis 90 % der suspendierten Teilchen eine Teilchengröße kleiner 30 µm [D(0.9) < 30 µm] hatten. Dies entsprach einer mittleren Partikelgröße D[4,3] von 15,3 µm.

Nach der Abtrennung von den Mahlkörpern wurde ein Teil der Dispersion mit der 10 fachen Menge Maiskeimöl verdünnt und über 12 h stehengelassen. Weder die unverdünnte noch die verdünnte Dispersion zeigten über diesen Zeitraum Sedimentationserscheinungen.

### Beispiel 4

Analog Beispiel 2 wurde eine Mischung aus

| Komponente | Menge in g |
|---|---|
| Calcium-d-pantothenat | 88 |
| Nicotinsäure | 320 |
| Folsäure | 2,4 |
| Biotin | 16 |
| Vitamin B₁₂ | 0,3 |
| Vitamin B₆-HCl | 32 |
| Vitamin B₂ | 50 |
| Vitamin B₁-HCl | 24 |
| Vitamin K₃ (Menadion-Na-Bisulfit) | 8 |
| Vitamin E-Acetat | 480 |
| Vitamin D₃ | 0,2 |
| Vitamin A-Acetat | 30 |
| Maiskeimöl | 600 |

suspendiert und durch eine kontinuierlich betriebene Kugelmühle gemahlen. Der Mahlvorgang wurde so lange durchgeführt, bis 90 % der suspendierten Teilchen eine Teilchengröße kleiner 20 µm [D(0.9) < 20 µm] hatten. Dies entsprach einer mittleren Partikelgröße D[4,3] von 10,9 µm.

Nach der Abtrennung von den Mahlkörpern wurde ein Teil der Dispersion mit der 10 fachen Menge Maiskeimöl verdünnt und über 12 h stehengelassen. Weder die unverdünnte noch die verdünnte Dispersion zeigten über diesen Zeitraum Sedimentationserscheinungen.

### Beispiel 5

Analog Beispiel 2 wurde eine Mischung aus

| Komponente | Menge in g |
|---|---|
| Vitamin C | 400 |
| Calcium-d-pantothenat | 88 |
| Nicotinsäure | 320 |
| Folsäure | 2,4 |
| Biotin | 1,6 |
| Vitamin B₁₂ | 0,3 |
| Vitamin B₆-HCl | 32 |
| Vitamin B₂ | 50 |
| Vitamin B₁-HCl | 24 |
| Vitamin K₃ (Menadion-Na-Bisulfit) | 8 |
| Sonnenblumenöl | 1000 |

suspendiert und durch eine kontinuierlich betriebene Kugelmühle gemahlen. Der Mahlvorgang wurde so lange durchgeführt, bis 90 % der suspendierten Teilchen eine Teilchengröße kleiner 20 µm [D(0.9) < 20 µm] hatten. Dies entsprach einer mittleren Partikelgröße D[4,3] von 10,9 µm.

Nach der Abtrennung von den Mahlkörpern wurde ein Teil der Dispersion mit der 10 fachen Menge Sonnenblumenöl verdünnt und über 12 h stehengelassen. Weder die unverdünnte noch die verdünnte Dispersion zeigten über diesen Zeitraum Sedimentationserscheinungen.

### Beispiel 6

Analog Beispiel 2 wurde eine Mischung aus

| Komponente | Menge in g |
|---|---|
| Vitamin C | 400 |
| Calcium-d-pantothenat | 88 |
| Nicotinsäure | 320 |
| Folsäure | 2,4 |
| Biotin | 1,6 |
| Vitamin B₁₂ | 0,3 |
| Vitamin B₆-HCl | 32 |
| Vitamin B₂ | 50 |
| Vitamin B₁-HCl | 24 |
| Vitamin K₃ (Menadion-Na-Bisulfit) | 8 |
| Fischöl | 1000 |

suspendiert und durch eine kontinuierlich betriebene Kugelmühle gemahlen. Der Mahlvorgang wurde so lange durchgeführt, bis 90 % der suspendierten Teilchen eine Teilchengröße kleiner 20 µm [D(0.9) < 20 µm] hatten. Dies entsprach einer mittleren Partikelgröße D[4,3] von 10,9 µm.

Nach der Abtrennung von den Mahlkörpern wurde ein Teil der Dispersion mit der 10 fachen Menge Fischöl verdünnt und über 12 h stehengelassen. Weder die unverdünnte noch die verdünnte Dispersion zeigten über diesen Zeitraum Sedimentationserscheinungen.

### Beispiel 7

Analog Beispiel 2 wurde eine Mischung aus

| Komponente | Menge in g |
|---|---|
| Vitamin C | 200 |
| Calcium-d-pantothenat | 140 |
| Nicotinsäureamid | 280 |
| Folsäure | 4,0 |
| Biotin | 2,0 |
| Vitamin B₁₂ | 0,1 |
| Vitamin B₆-HCl | 20 |
| Vitamin B₂ | 100 |
| Vitamin B₁-HCl | 40 |
| Vitamin K₃ (Menadion-Na-Bisulfit) | 20 |
| Vitamin E-Acetat | 400 |
| Vitamin D₃ | 0,10 |
| Vitamin A-Acetat | 8,2 |
| D,l-α-Tocopherol | 600 |

suspendiert und durch eine kontinuierlich betriebene Kugelmühle gemahlen. Der Mahlvorgang wurde so lange durchgeführt, bis 90 % der suspendierten Teilchen eine Teilchengröße kleiner 20 µm [D(0.9) < 20 µm] hatten. Dies entsprach einer mittleren Partikelgröße D[4,3] von 10,9 µm.

Nach der Abtrennung von den Mahlkörpern wurde ein Teil der Dispersion mit der 10 fachen Menge Maiskaimöl verdünnt und über 12 h stehengelassen. Weder die unverdünnte noch die verdünnte Dispersion zeigten über diesen Zeitraum Sedimentationserscheinungen.

### Beispiel 8

Analog Beispiel 2 wurde eine Mischung aus

| Komponente | Menge in g |
|---|---|
| Vitamin C | 100 |
| Lucantin® Pink (10%iges Astaxanthin Trockenpulver, Fa. BASF) | 500 |
| Vitamin K₃ (Menadion-Na-Bisulfit) | 5 |
| Vitamin B₁ Nitrat | 15 |
| Vitamin A-Acetat | 5,0 |
| Vitamin E-Acetat | 50 |
| Ethoxyquin | 0,1 |
| Fischöl | 1000 |

suspendiert und durch eine kontinuierlich betriebene Kugelmühle gemahlen. Der Mahlvorgang wurde so lange durchgeführt, bis 90 % der suspendierten Teilchen eine Teilchengröße kleiner 20 µm [D(0.9) < 20 µm] hatten.

Nach der Abtrennung von den Mahlkörpern wurde ein Teil der Dispersion mit der 10 fachen Menge Fischöl verdünnt und über 12 h stehengelassen. Weder die unverdünnte noch die verdünnte Dispersion zeigten über diesen Zeitraum Sedimentationserscheinungen.

### Beispiel 9

Analog Beispiel 2 wurde eine Mischung aus

| Komponente | Menge in g |
|---|---|
| Vitamin C | 200 |
| β-Carotin (krist.) | 10 |
| Calcium-d-pantothenat | 140 |
| Nicotinsäureamid | 280 |
| Folsäure | 4,0 |
| Biotin | 2,0 |
| Vitamin B₁₂ | 0,1 |
| Vitamin B₆-HCl | 20 |
| Vitamin B₂ | 100 |
| Vitamin B₁-HCl | 40 |
| Vitamin K₃ (Menadion-Na-Bisulfit) | 20 |
| Vitamin E-Acetat | 400 |
| Vitamin D₃ | 0,10 |
| Vitamin A-Acetat | 8,2 |
| D,1-α-Tocopherol | 600 |

suspendiert und durch eine kontinuierlich betriebene Kugelmühle gemahlen. Der Mahlvorgang wurde so lange durchgeführt, bis 90 % der suspendierten Teilchen eine Teilchengröße kleiner 20 µm [D(0.9) < 20 µm] hatten.

Nach der Abtrennung von den Mahlkörpern wurde ein Teil der Dispersion mit der 10 fachen Menge Maiskeimöl verdünnt und über 12 h stehengelassen. Weder die unverdünnte noch die verdünnte Dispersion zeigten über diesen Zeitraum Sedimentationserscheinungen.

### Beispiel 10

Analog Beispiel 4 wurde eine Mischung aus

| Komponente | Menge in g |
|---|---|
| Calcium-d-pantothenat | 88 |
| Nicotinsäure | 320 |
| Folsäure | 2,4 |
| Biotin | 16 |
| Vitamin B₁₂ | 0,3 |
| Vitamin B₆-HCl | 32 |
| Vitamin B₂ | 50 |
| Vitamin B₁-HCl | 24 |
| Vitamin K₃ (Menadion-Na-Bisulfit) | 8 |
| Vitamin E-Acetat | 480 |
| Vitamin D₃ | 0,2 |
| Vitamin A-Acetat | 30 |
| CaCl₂ (wasserfrei) | 100 |
| Maiskeimöl | 600 |

suspendiert und durch eine kontinuierlich betriebene Kugelmühle gemahlen. Der Mahlvorgang wurde so lange durchgeführt, bis 90 % der suspendierten Teilchen eine Teilchengröße kleiner 20 µm [D(0.9) < 20 µm] hatten. Dies entsprach einer mittleren Partikelgröße D[4,3] von 10,9 µm.

Nach der Abtrennung von den Mahlkörpern wurde ein Teil der Dispersion mit der 10 fachen Menge Maiskeimöl verdünnt und über einen Zeitraum von einem Monat bei 40°C gelagert. Weder die unverdünnte noch die verdünnte Dispersion zeigten über diesen Zeitraum Sedimentationserscheinungen.

## Patentansprüche

1. Verfahren zur Herstellung öliger Suspensionen wasserlöslicher Vitamine, **dadurch gekennzeichnet, daß** man
a) mindestens ein wasserlösliches Vitamin in einem Öl bis auf eine mittlere Partikelgröße von 0,1 bis 100 µm mahlt oder
b) mindestens ein wasserlösliches Vitamin ohne Verwendung einer kontinuierlichen Phase bis auf eine mittlere Partikelgröße von 0,1 bis 100 µm mahlt und die gemahlenen Partikel anschließend in einem Öl suspendiert.

2. Verfahren nach Anspruch 1, **dadurch gekennzeichnet, daß** es sich bei dem Öl um mindestens ein eßbares Öl handelt.

3. Verfahren nach einem der Ansprüche 1 oder 2, **dadurch gekennzeichnet, daß** es sich bei dem Öl um ein bei 20°C flüssiges Öl handelt.

4. Verfahren nach einem der Ansprüche 2 oder 3, **dadurch gekennzeichnet, daß** man als eßbares Öl Vitamin E, Vitamin E-Derivate oder Mischungen davon verwendet.

5. Verfahren nach einem der Ansprüche 1 bis 4, **dadurch gekennzeichnet, daß** die Mahlung im Schritt a) sowie die Mahlung und/oder Suspendierung in Schritt b) in Abwesenheit eines Emulgators erfolgen.

6. Verfahren nach einem der Ansprüche 1 bis 5, **dadurch gekennzeichnet, daß** die Mahlung im Schritt a) sowie die Mahlung und/oder Suspendierung in Schritt b) in Abwesenheit eines Schutzkolloids erfolgen.

7. Verfahren nach einem der Ansprüche 1 bis 6, **dadurch gekennzeichnet, daß** man mindestens eines der wasserlöslichen Vitamine in kristalliner Form einsetzt.

8. Verfahren nach einem der Ansprüche 1 bis 7, **dadurch gekennzeichnet, daß** die Mahlung im Schritt a) sowie die Suspendierung in Schritt b) in Gegenwart fettlöslicher Vitamine erfolgen.

9. Verfahren nach einem der Ansprüche 1 bis 8, **dadurch gekennzeichnet, daß** die Mahlung im Schritt a) sowie die Mahlung und/oder Suspendierung in Schritt b) in Gegenwart von Trokkenmitteln erfolgen.

10. Verfahren nach Anspruch 9, **dadurch gekennzeichnet, daß** man als Trockenmitteln Stoffe, ausgewählt aus der Gruppe, bestehend aus Alkali- und Erdalkalisulfaten, Alkali- und Erdalkalichloriden und Kieselgel verwendet.

11. Ölige Suspensionen mindestens eines wasserlöslichen Vitamins, erhältlich nach einem Verfahren, definiert gemäß einem der Ansprüche 1 bis 10.

12. Ölige Suspensionen nach Anspruch 11, enthaltend 5 bis 70 Gew.-% mindestens eines wasserlöslichen Vitamins.

13. Ölige Suspensionen nach einem der Ansprüche 11 oder 12, enthaltend zusätzlich 0,5 bis 60 Gew.-% mindestens eines fettlöslichen Vitamins.

14. Verwendung der öligen Suspensionen, definiert gemäß einem der Ansprüche 11 bis 13 als Zusatz zu Lebensmitteln und Tierfuttermitteln, Pharmazeutika und kosmetischen Zubereitungen.

15. Verwendung nach Anspruch 14 als Futtermittelzusatz in der Tierernährung.

16. Verwendung nach Anspruch 15 zum Auftragen auf Futtermittelpellets.

17. Verwendung nach Anspruch 16, **dadurch gekennzeichnet, daß** man die Futtermittelpellets unter vermindertem Druck mit der öligen Suspension belädt.

18. Futtermittelpellets, enthaltend ölige Suspensionen, definiert gemäß einem der Ansprüche 11 bis 13.

## Claims

1. A process for producing oily suspensions of water-soluble vitamins, which comprises
a) grinding at least one water-soluble vitamin in an oil until the average particle size is from 0.1 to 100 µm or
b) grinding at least one water-soluble vitamin without using a continuous phase until the average particle size is from 0.1 to 100 µm and subsequently suspending the ground particles in an oil.

2. A process as claimed in claim 1, wherein the oil is at least one edible oil.

3. A process as claimed in either of claims 1 and 2, wherein the oil is an oil which is liquid at 20°C.

4. A process as claimed in either of claims 2 and 3, wherein vitamin E, vitamin E derivatives or mixtures thereof are used as edible oil.

5. A process as claimed in any of claims 1 to 4, wherein the grinding in step a) and the grinding and/or suspending in step b) take place in the absence of an emulsifier.

6. A process as claimed in any of claims 1 to 5, wherein the grinding in step a) and the grinding and/or suspending in step b) take place in the absence of a protective colloid.

7. A process as claimed in any of claims 1 to 6, wherein at least one of the water-soluble vitamins is employed in crystalline form.

8. A process as claimed in any of claims 1 to 7, wherein the grinding in step a) and the suspending in step b) take place in the presence of lipid-soluble vitamins.

9. A process as claimed in any of claims 1 to 8, wherein the grinding in step a) and the grinding and/or suspending in step b) take place in the presence of desiccants.

10. A process as claimed in claim 9, wherein substances selected from the group consisting of alkali metal and alkaline earth metal sulfates, alkali metal and alkaline earth metal chlorides and silica gel are used as desiccants.

11. An oily suspension of at least one water-soluble vitamin obtainable by a process as defined in any of claims 1 to 10.

12. An oily suspension as claimed in claim 11, comprising from 5 to 70% by weight of at least one water-soluble vitamin.

13. An oily suspension as claimed in either of claims 11 and 12, additionally comprising from 0.5 to 60% by weight of at least one lipid-soluble vitamin.

14. The use of the oily suspensions as defined in any of claims 11 to 13 as addition to human foods and animal feeds, pharmaceuticals and cosmetic preparations.

15. The use as claimed in claim 14 as feed additive in livestock nutrition.

16. The use as claimed in claim 15 for application to feed pellets.

17. The use as claimed in claim 16, wherein the feed pellets are loaded with the oily suspension under reduced pressure.

18. Feed pellets comprising oily suspensions as defined in any of claims 11 to 13.

## Revendications

1. Procédé de préparation de suspensions huileuses de vitamines solubles dans l'eau, **caractérisé en ce que**
a) on broie au moins une vitamine soluble dans l'eau dans une huile jusqu'à une taille moyenne de particule de 0,1 à 100 µm ou
b) on broie au moins une vitamine soluble dans l'eau sans utilisation d'une phase continue jusqu'à une taille moyenne de particule de 0,1 à 100 µm et on met ensuite les particules broyées en suspension dans une huile.

2. Procédé suivant la revendication 1, **caractérisé en ce que**, en ce qui concerne l'huile, il s'agit d'au moins une huile comestible.

3. Procédé suivant l'une des revendications 1 et 2, **caractérisé en ce que**, en ce qui concerne l'huile, il s'agit d'une huile fluide à 20°C.

4. Procédé suivant l'une des revendications 2 et 3, **caractérisé en ce que**, comme huile comestible, on utilise de la vitamine E, des dérivés de vitamine E ou leurs mélanges.

5. Procédé suivant l'une des revendications 1 à 4, **caractérisé en ce que** le broyage dans l'étape a) ainsi que le broyage et/ou la mise en suspension dans l'étape b) ont lieu en l'absence d'un agent émulsionnant.

6. Procédé suivant l'une des revendications 1 à 5, **caractérisé en ce que** le broyage dans l'étape a) ainsi que le broyage et/ou la mise en suspension dans l'étape b) ont lieu en l'absence d'un colloïde de protection.

7. Procédé suivant l'une des revendications 1 à 6, **caractérisé en ce qu'**on met en oeuvre au moins une des vitamines solubles dans l'eau sous forme cristalline.

8. Procédé suivant l'une des revendications 1 à 7, **caractérisé en ce que** le broyage dans l'étape a) ainsi que la mise en suspension dans l'étape b) ont lieu en présence de vitamines liposolubles.

9. Procédé suivant l'une des revendications 1 à 8, **caractérisé en ce que** le broyage dans l'étape a) ainsi que le broyage et/ou la mise en suspension dans l'étape b) ont lieu en présence d'agents desséchants.

10. Procédé suivant la revendication 9, **caractérisé en ce que**, comme agents desséchants, on utilise des substances choisies parmi le groupe constitué de sulfates de métal alcalin et de métal alcalino-terreux, de chlorures de métal alcalin et de métal alcalino-terreux et de gel de silice.

11. Suspensions huileuses d'au moins une vitamine soluble dans l'eau, que l'on peut obtenir suivant un procédé défini conformément à une des revendications 1 à 10.

12. Suspensions huileuses suivant la revendication 11, contenant 5 à 70 % en poids d'au moins une vitamine soluble dans l'eau.

13. Suspensions huileuses suivant une des revendications 11 et 12, contenant en supplément 0,5 à 60 % en poids d'au moins une vitamine liposoluble.

14. Utilisation des suspensions huileuses, définies conformément à une des revendications 11 à 13, comme additif à des aliments et des fourrages, des produits pharmaceutiques et des compositions cosmétiques.

15. Utilisation suivant la revendication 14, comme additif pour fourrage dans l'alimentation des animaux.

16. Utilisation suivant la revendication 15 pour l'application sur des pellets de fourrage.

17. Utilisation suivant la revendication 16, **caractérisée en ce qu'**on charge les pellets de fourrage avec la suspension huileuse sous une pression réduite.

18. Pellets de fourrage contenant des suspensions huileuses définies conformément à une des revendications 11 à 13.
